# EUROPÄISCHE PATENTSCHRIFT

(11) **EP 0 892 620 B1**
(45) Veröffentlichungstag und Bekanntmachung des Hinweises auf die Patenterteilung: **27.11.2002**
(21) Anmeldenummer: 98909408.1
(22) Anmeldetag: 06.02.1998
(51) Int. Cl.: A61B 17/04

(54) **CHIRURGISCHER FADENSCHNEIDER**
SURGICAL THREAD CUTTER
COUPE-FILS CHIRURGICAL

(30) Priorität: 07.02.1997 DE 19704580
(43) Veröffentlichungstag der Anmeldung: 27.01.1999
(73) Patentinhaber: Karl Storz GmbH & Co. KG, 78532 Tuttlingen (DE)
(72) Erfinder: KOH, Charles, Memequon, WI 53092 (US); DITTRICH, Horst, D-78194 Immendingen (DE); SOLINGEN, Simon, Los Angeles, CA 90077 (US)
(74) Vertreter: Weller, Wolfgang, Dr.rer.nat.
(86) Internationale Anmeldenummer: EP9800669
(87) Internationale Veröffentlichungsnummer: WO98034545

(56) Entgegenhaltungen:
- US-A- 3 372 477
- US-A- 3 802 074
- US-A- 5 549 623

## Beschreibung

Die Erfindung betrifft einen chirurgischen Fadenschneider mit einer über einen Betätigungsmechanismus relativ auf einen Amboß hin- und von diesem wegbewegbaren Klinge, sowie mit einer Feder, über die die Klinge in einer Bewegungsrichtung mit Federkraft beaufschlagbar ist.

Derartige chirurgische Fadenschneider werden dafür herangezogen, um ein Fadenmaterial, das für intrakorporale Nähte verwendet wird, zu schneiden. Es wurde festgestellt, daß mit den normalerweise für den Gewebeschnitt benutzten Präparier-Scheren das Fadenmaterial nur schwer geschnitten werden kann. Insbesondere wurde festgestellt, daß sich die Fäden mit den Scheren verklemmen.

Aus dem DE 92 14 580.9 U1 ist ein chirurgischer Fadenschneider bekannt geworden, der eine scharfe Klinge aufweist, die sich gegen einen flachen Amboß bewegt und damit den Faden schneidet. Dazu sind Klinge und Amboß relativ zueinander beweglich, die Bewegung wird über solche Griffteile gesteuert, wie sie üblicherweise bei medizinischen Scheren eingesetzt werden und die über ein Scharnier miteinander verbunden sind. Der Amboß weist einen Schlitz auf, in den die scharfe Klinge einfahren kann und so den gegen den Amboß gedrückten Faden durchtrennt.

Aus dem Firmenprospekt der Anmelderin "Endoskopische Chirurgie, 2. Ausgabe 1/94, Kapitel 8, Naht und Ligatur, Seite NH 4 A" ist ein chirurgischer Fadenschneider nach dem eingangs erwähnten Prinzip von Scarfi bekannt, bei dem die Klinge in einem Schaft hin- und herbewegbar aufgenommen ist, an dessen distalem Ende der Amboß angeordnet ist. Durch den Schaft führt ein mit der Klinge verbundenes stabförmiges Betätigungselement und dieses ist am proximalen Ende mit einem ringförmigen Griffteil verbunden. Am proximalen Ende erstreckt sich parallel zum Schaft, also auch parallel zur Schaftachse eine Feder, die sich einerseits an dem ringförmigen Griffteil am anderen Ende an einem weiteren, am Schaft ortsfest angebrachten Griffteil abstützt. Die Feder ist derart vorgespannt, daß das ringförmige Griffteil samt Betätigungselement und daran angebrachter Klinge vom Amboß weg gedrückt werden, somit zwischen der Schneide der Klinge und dem Amboß Raum vorhanden ist, um den zu durchtrennenden Faden zwischen Klinge und Amboß bringen zu können. Zum Durchtrennen des Fadens wird das distale Ende des Fadenschneiders so an den Faden gebracht, daß dieser zwischen Klinge und Amboß zum Liegen kommt, wobei der Fadenschneider selbst bspw. durch einen Trokar in den Körper eingeführt ist. Zum Durchtrennen des Fadens wird das ringförmige Griffteil am proximalen Ende relativ zum ortsfesten Griffteil gegen die Kraft der Feder nach distal verschoben, wobei die Klinge auf den Amboß zu bewegt wird und den dazwischen aufgenommenen Faden durchtrennt.

Aus der DE 42 04 051 C2 ist eine Amboßschere für chirurgische Zwecke bekannt, bei der die Klinge derart angeordnet ist, daß in Abhängigkeit von der Fadenstärke und dem Schneidegut der ziehende Schnitt umso stärker ist, je mehr Widerstand das Schneidegut dem Schließen der Klinge entgegensetzt. Die Klinge ist dabei um eine Achse verschwenkbar und auf den Amboß zu und von diesem weg bewegbar.

Aus dem DE 91 09 097 U1 ist eine Haltezange bekannt, die eine Feder enthält, mit der eine Vorspannung erzeugt wird, um die beiden Zangenarme in Verschlußrichtung zu beaufschlagen. Es handelt sich dabei um eine Fadenhaltevorrichtung. Eine Arretiervorrichtung dient dazu, um einen Zangenarm zu verschieben.

Aus dem DE 91 12 301 U1 ist ein Nadelhalter bekannt, der einen Stab aufweist, der von einer axial relativ zum Stab bewegbaren Hülse umgeben ist. Die Hülse ist verschiebbar und durch eine Feder gegen eine radiale Anlagefläche vorgespannt.

Die Dokumente US 3 802 074, US 3 328 876 und US 3 372 477 betreffen Fadenschneider, bei denen die Klinge längs eines schaftartigen Teiles hin- und herbewegt werden kann, und zwar durch die Hand bzw. einen Finger der Betätigungsperson, die den Fadenschneider in der Hand hält. Die Schneidebewegung und die Schneidewirkung wird somit durch die jeweils individuelle Handkraft und Fingerfertigkeit der Bedienungsperson bestimmt.

Im praktischen Einsatz wurde festgestellt, daß es vorkommen kann, daß die Klinge den Faden gegen den Amboß drückt und diesen, ohne ihn zu durchtrennen, in den Schlitz im Amboß einschiebt anstatt den Faden zu durchtrennen. Dabei kann sich die Klinge derart mit dem Faden verklemmen, daß die Kraft der Feder nicht ausreichend ist, um die Klinge wieder vom Amboß abzuziehen.

Ferner wurde nachteilig festgestellt, daß es wesentlich von der Geschicklichkeit der Bedienungsperson abhängt, ob schon mit einer einzigen Klingenbewegung der Faden durchtrennt werden kann oder ob dazu mehrere Bewegungen notwendig sind. Auf jeden Fall wurde festgestellt, daß eine hohe Aufmerksamkeit beim Durchtrennen des Fadens notwendig ist.

Es ist nun Aufgabe der vorliegenden Erfindung, hier Abhilfe zu schaffen und einen chirurgischen Fadenschneider der eingangs genannten Art dahingehend weiterzuentwickeln, daß dieser funktionssicher arbeitet, insbesondere mit einem einzigen Schnittversuch sofort den Faden durchtrennt.

Erfindungsgemäß wird die Aufgabe dadurch gelöst, daß die Feder die Klinge in Schneiderichtung beaufschlagt, daß eine Arretierung vorgesehen ist, über die die Klinge in einer vom Amboß entfernten Position feststellbar ist, und daß die Kraft der Feder derart eingestellt ist, daß die Klinge, nach Lösen der Arretierung, guillotineartig auf den Amboß zubewegbar ist.

Ist die Arretierung gelöst, hat die Feder die Klinge in den Amboß hineingedrückt. Dies stellt eine definierte Endposition der Klinge dar, in der während der Verbringung des distalen Endes an die zu schneidende Stelle im Körper keine Gewebeteile oder dgl. zwischen die Klinge und Amboß treten können. Zur Vorbereitung des Schneidevorganges wird die Klinge über den Betätigungsmechanismus gegen die Kraft der Feder vom Amboß weg bewegt und in dieser Position arretiert. Nunmehr kann der Faden zwischen Klinge und Amboß gebracht werden. Die Kraft der gespannten Druckfeder ist nun so eingestellt, daß nach Lösen der Arretierung die Klinge sehr schnell, also guillotineartig auf den Amboß zu bewegt wird. Diese rasche Bewegung mit der Unterstützung der entsprechenden Druckkraft der Feder sorgt nun dafür, daß der Faden sofort von der Klinge durchtrennt wird und nicht von dieser mitgerissen oder gar in den Schlitz im Amboß hineingedrückt wird. Der Faden stellt, obwohl er nur eine geringe Masse hat, gegenüber der sehr schnell bewegten Klinge eine Ruhemasse dar, so daß die den Faden erreichende Klinge sich sofort in dessen Material hineingräbt, bevor dieser auch aus seiner Ruhelage bewegt wird. Dadurch ist schon eine Schnittansatzstelle geschaffen, die den weiteren Schnittvorgang definitiv ablaufen läßt, den Faden also durchtrennt, ohne daß die beidseits der Klinge liegenden Fadenenden bewegt, also von der Klinge mitgerissen werden. Wurde mit einem solchen Faden bspw. eine Wunde genäht, führt ein solch rasches Abtrennen, ohne daß Zugkräfte am Faden wirken, zu keiner Beeinträchtigung der genähten Operationsstelle. Nach dem Schneidevorgang ist die Klinge in den Amboß eingefahren, und der Fadenschneider kann vom Körper abgezogen werden, ohne die Gefahr, daß sich in den muldenartigen Bereich zwischen Klinge und Amboß Gewebeteile verfangen und ggf. das Gewebe beschädigt wird. Die Feder kann proximal oder distal angeordnet sein, je nach den baulichen Gegebenheiten.

Somit wird die Aufgabe vollkommen gelöst.

In einer weiteren Ausgestaltung der Erfindung ist der Betätigungsmechanismus längs der Bewegungsrichtung der Klinge bewegbar, und er ist relativ zur Bewegungsrichtung drehbar und in einer verdrehten Stellung durch die Arretierung arretierbar.

Die an sich bekannte Maßnahme, daß der Betätigungsmechanismus längs der Bewegungsrichtung der Klinge bewegbar ist, hat den Vorteil, daß beim Bewegen der Klinge keine Kippmomente auf den Fadenschneider ausgeübt werden, wie das bspw. bei den eingangs genannten Scherengriffteilen mit Scharnierverbindung der Fall ist. Durch die Verdrehbarkeit und Arretierbarkeit kann der Betätigungsmechanismus, nachdem die Klinge vom Amboß abgezogen wurde, durch einfaches Verdrehen arretiert werden bzw. durch eine entgegengesetzte Drehbewegung die Arretierung wieder gelöst werden. Solche Bewegungen sind einfach und ohne hohe Aufmerksamkeit mit einer Hand durchzuführen, so daß die Aufmerksamkeit des Operateurs am distalen Ende des Fadenschneiders konzentriert bleibt kann, um den abzuschneidenden bzw. durchzutrennenden Faden in einer geeigneten Position zwischen Amboß und Klinge zu halten.

In einer weiteren Ausgestaltung der Erfindung ist der Betätigungsmechanismus proximal in einem griffartigen Gehäuse aufgenommen.

Diese Maßnahme hat den Vorteil, daß der Fadenschneider über das Gehäuse ergriffen werden kann und von Fingern der Hand, die das Gehäuse ergriffen hat, kann dann der Betätigungsmechanismus bewegt werden. Dies erlaubt eine einfache Einhandbedienung.

In einer weiteren Ausgestaltung der Erfindung weist die Arretierung zumindest ein vom Betätigungsmechanismus radial vorspringendes Betätigungselement auf, das durch eine Bajonettführung im Gehäuse reicht.

Diese Maßnahme hat nun den erheblichen konstruktiven Vorteil, daß das Gehäuse nicht nur den Bewegungsmechanismus schützend umgibt, sondern auch zugleich konstruktiv einfach als Bauelement der Arretierung ausgebildet ist. Der zumindest eine radial vorstehende Betätigungsstift kann von einem Finger der Hand, die das Griffteil hält, entgegen der Kraft der Feder bewegt werden, wobei die Bajonettführung diese Bewegung eindeutig steuert. Durch ein geringfügiges umfängliches Verdrehen des Betätigungselementes in eine seitliche Ausnehmung der Bajonettführung kann dann der Betätigungsmechanismus in dieser Stellung arretiert werden. Es genügt dann ein sanfter Fingerdruck, um den Betätigungsstift wieder zurück verschwenken und die guillotineartige Bewegung der Klinge Richtung Amboß freizugeben. Die Vorgänge Spannen, Arretieren und Lösen der Arretierung sind ohne besondere Aufmerksamkeit durchzuführen und können ohne visuelle Aufmerksamkeit durch reines Ertasten und Bewegen des Betätigungselementes durchgeführt werden.

In einer weiteren Ausgestaltung der Erfindung sind zwei diametral gegenüberliegende, radial vorspringende Betätigungsstifte vorgesehen.

Diese Maßnahme hat den Vorteil, daß einerseits einfach ausgestaltete Bauelemente vorgesehen sind, die bspw. durch zwei Finger umgriffen und zum Steuern des Betätigungsmechanismus einfach bewegt werden können. Zum Spannen des Betätigungsmechanismus brauchen die beiden Stifte lediglich zurückgezogen und dann etwas verdreht werden, um den Arretierungsmechanismus festzustellen.

In einer weiteren Ausgestaltung der Erfindung ist die Bajonettführung als T-förmige Schlitzöffnung im Gehäuse ausgebildet.

Die Querbereiche des T ermöglichen nunmehr die Betätigungsstifte, nachdem diese längs des langen Teils des T zum Spannen der Feder verschoben wurden, entweder nach links oder nach rechts in einen Querbereich des T einzuschieben, je nachdem, wie es für die Bedienungsperson günstig ist oder diese es wünscht. Es ist also keine zusätzliche Aufmerksamkeit dahingehend aufzuwenden, ob nach Spannen des Betätigungsmechanismus nunmehr die Betätigungsstifte in oder entgegengesetzt dem Uhrzeigersinn verdreht werden müssen.

In einer weiteren Ausgestaltung der Erfindung sind die Querbereiche der T-förmigen Schlitzöffnungen, die sich in umfänglicher Richtung des Gehäuses erstrecken, in Richtung des Amboß etwas geneigt.

Diese Maßnahme hat nun den Vorteil, daß durch die geneigte Anordnung in Richtung Amboß die Betätigungsstifte aufgrund der Kraft der Feder an die geneigte distale Flanke der Nut gepreßt und geringfügig in Richtung Amboß bewegt werden. Beim Lösen der Arretierung müssen die Stifte nicht nur verdreht sondern dabei wieder etwas vom Amboß zurückgezogen werden, was die distale Flanke des Schlitzes bewerkstelligt. Dadurch ist ein Verschieben bzw. Verdrehen der Betätigungsstifte aus der arretierenden Stellung mit etwas mehr Kraftaufwand durchzuführen als bei exakt rechtwinklig T-förmiger Schlitzöffnung. Dadurch wird verhindert, daß schon ein versehentliches Berühren der Stifte die Arretierung löst.

In einer weiteren Ausgestaltung der Erfindung ist die Feder im griffartigen Gehäuse angeordnet.

Diese Maßnahme hat den Vorteil, insbesondere zusammen mit der Maßnahme, daß auch der weitere Betätigungsmechanismus im Griff angeordnet ist, daß diese Bauteile geschützt im Gehäuse aufgenommen sind, was ebenfalls zur Betriebssicherheit beiträgt.

In einer weiteren Ausgestaltung der Erfindung ist ein Dämpfungsglied vorgesehen, das die Bewegung der Klinge am Ende der Schneidebewegung dämpft.

Dabei ist insbesondere vorgesehen, daß der Betätigungsmechanismus ein Dämpfungsglied aufweist, durch das der Aufprall des Betätigungsmechanismus an einem Anschlag am Ende der Schneidebewegung gedämpft ist.

Diese Maßnahme hat den Vorteil, daß am Ende des Bewegungsvorganges, also nachdem der Faden durchgetrennt ist, der Aufprall des Betätigungselementes auf einen Anschlag gedämpft ist, so daß nicht die Gefahr besteht, daß der Fadenschneider durch den Aufpralldruck bzw. -ruck ungünstig in den Patienten hinein bewegt wird.

In einer weiteren Ausgestaltung der Erfindung ist im Gehäuse ein elastisches Dämpfungsglied vorgesehen, das gegen eine innere Wand des Gehäuses prallt und dabei verformbar ist.

Diese Maßnahme hat den Vorteil, daß auch das Dämpfungsglied im Gehäuse aufgenommen ist, so daß nicht nur der mechanische Aufprall gedämpft ist sondern auch das Aufprallgeräusch gedämpft ist. Das elastische Element ist auch durch das Gehäuse gegen mechanische Beeinträchtigung geschützt.

In einer weiteren Ausgestaltung der Erfindung ist die Klinge in einem Schaft aufgenommen und Schaft und Klinge sind voneinander lösbar.

Diese Maßnahme hat den Vorteil, daß die Klinge einerseits geschützt und sicher geführt in dem Schaft aufgenommen ist und daß zu Reinigungszwecken Klinge und Schaft voneinander gelöst werden können.

In einer weiteren Ausgestaltung der Erfindung weist der Amboß die Form eines Hakens auf.

Diese Maßnahme hat den Vorteil, daß durch die Ausbildung des Amboß als Haken der zu durchtrennende Faden mit dem Amboß ergriffen und in die richtige Position gebracht bzw. gezogen werden kann. So ist es auch möglich, wenn bspw. ein Fadenende abgetrennt werden soll, dies mit dem Haken in eine für den Operateur günstige, sprich visuell ersichtliche Position zu bringen, bspw. in den Blickbereich eines Endoskopes, um dann den Schneidevorgang kontrolliert durchführen zu können.

In einer weiteren Ausgestaltung der Erfindung ist eine Spitze des Hakens einer Spitze der Klinge zugewandt.

Diese Maßnahme hat den Vorteil, daß die sich relativ aufeinander zu bewegenden Spitzen alsbald den inneren Hakenraum abschließen, nämlich nachdem die Spitze der Klinge auf Höhe der Spitze des Amboß gelangt ist, so daß der Faden dann unverlierbar gefangen ist und nicht mehr versehentlich aus dem Hakenraum vor dem Amboß heraustreten kann. Dadurch wird ein besonders sicheres Schneiden des Fadens bewirkt.

In einer weiteren Ausgestaltung der Erfindung ist der Amboß Teil des Schaftes und der Schaft ist mit dem griffartigen Gehäuse lösbar verbindbar.

Diese Maßnahme hat den Vorteil, daß wenige Bauteile notwendig sind, die zum Reinigen einfach zerlegt werden können.

In einer weiteren Ausgestaltung der Erfindung weist der Amboß einen Längsschlitz auf, in den die Klinge einfahrbar ist.

Diese Maßnahme hat den Vorteil, daß die Klinge sicher geführt in den Amboß einfahren kann und dort in der entspannten Endstellung verbleibt. Dadurch ist auch sichergestellt, daß die freie Spitze der Klinge geschützt im Amboß aufgenommen ist, so daß Verletzungen von Handhabungspersonen nach Durchführen des Schnittes ausgeschlossen sind.

In einer weiteren Ausgestaltung der Erfindung läuft die Klinge auf eine Fläche des Amboß auf.

Diese Maßnahme hat den Vorteil, daß die Klinge den zu zerschneidenden Faden mitnehmen kann und dann beim Auftreffen auf die Fläche des Amboß zertrennt.

In einer weiteren Ausgestaltung der Erfindung weist die Klinge eine Schneidekante auf, die mit einer Kante des Amboß zusammenwirkt.

Diese Maßnahme hat den Vorteil, daß die Klinge und die Kante des Amboß wie zwei Scherenteile miteinander wirken, so daß ein scherenartiger Schnitt bewerkstelligt wird, was bei manchen Fadenmaterialien von Vorteil ist.

In einer weiteren Ausgestaltung der Erfindung ist die Länge des Laufweges der Klinge einstellbar.

Diese Maßnahme hat den erheblichen Vorteil, daß die Beschleunigungsstrecke der Klinge je nach Fadenstärke, Fadenart oder auch zum Ausgleich von Ermüdungen der Feder variabel einstellbar ist.

In einer weiteren Ausgestaltung der Erfindung ist die Feder als Schraubenfeder, als Luftdruckfeder, oder als magnetisch gespannte Feder ausgebildet.

Diese Federausgestaltungen erlauben schlanke Bauweisen, die in dem Griffteil untergebracht werden können und erlauben den guillotineartigen Vortrieb der Klinge auf besonders vorteilhafte Weise.

In einer weiteren Ausgestaltung der Erfindung ist der chirurgische Fadenschneider Teil eines multifunktionalen Instrumentes.

In dieser Ausgestaltung können die Vorgänge Nähen und Auftrennen des genähten Fadens mit einem einzigen multifunktionalen Instrument durchgeführt werden, ohne daß nacheinander verschiedene Instrumente an die Operationsstelle verbracht werden müssen bzw. ein Instrument zum Nähen vorher abgezogen werden muß, bevor ein Fadenschneider eingesetzt werden kann.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nachfolgend anhand eines ausgewählten Ausführungsbeispiels näher beschrieben und erläutert. Es zeigen:
- Fig. 1: eine Seitenansicht eines erfindungsgemäßen Fadenschneiders, bei dem die Klinge in den Amboß eingefahren ist;
- Fig. 2: einen Längsschnitt des Fadenschneiders von Fig. 1, in einer um 90 um dessen Längsachse verdrehten Stellung;
- Fig. 2a: eine Seitenansicht lediglich des distalen Endes in einem Zwischenzustand, in dem die Klinge vom Amboß weg bewegt ist; und
- Fig. 2b: eine der Fig. 2a entsprechende Darstellung im vollständig zurückgezogenen Zustand der Klinge.

Ein in den Figuren in seiner Gesamtheit mit der Bezugsziffer 10 bezeichneter erfindungsgemäßer Fadenschneider weist ein langerstrecktes stabförmiges Gehäuse 12 auf, von dem ein relativ langer zylindrischer Schaft 14 vorspringt. Ein im Inneren des hohlen Schaftes 14 aufgenommenes Betätigungselement 16 ist am distalen Ende mit einer scharfen Klinge 18 verbunden.

Am distalen Ende des Schaftes 14 ist ein Amboß 20 vorgesehen, der mittig über einen Längsschlitz 22 geteilt ist, in den die schmale Klinge 18 passend einschiebbar ist.

In der Wand des Gehäuses 12 ist eine Bajonettführung 24 in Form einer T-Schlitzöffnung 26 vorgesehen.

Der langerstreckte Bereich des "T" erstreckt sich längs der Längsachse des Schaftes 14 bzw. der Längsachse des Gehäuses 12, und der Querbereich 27 liegt proximal und erstreckt sich in umfänglicher Richtung des Gehäuses 12.

Die Bajonettführung 24 ist Bauelement einer Arretierung 28 für einen im Gehäuse aufgenommenen Betätigungsmechanismus 30.

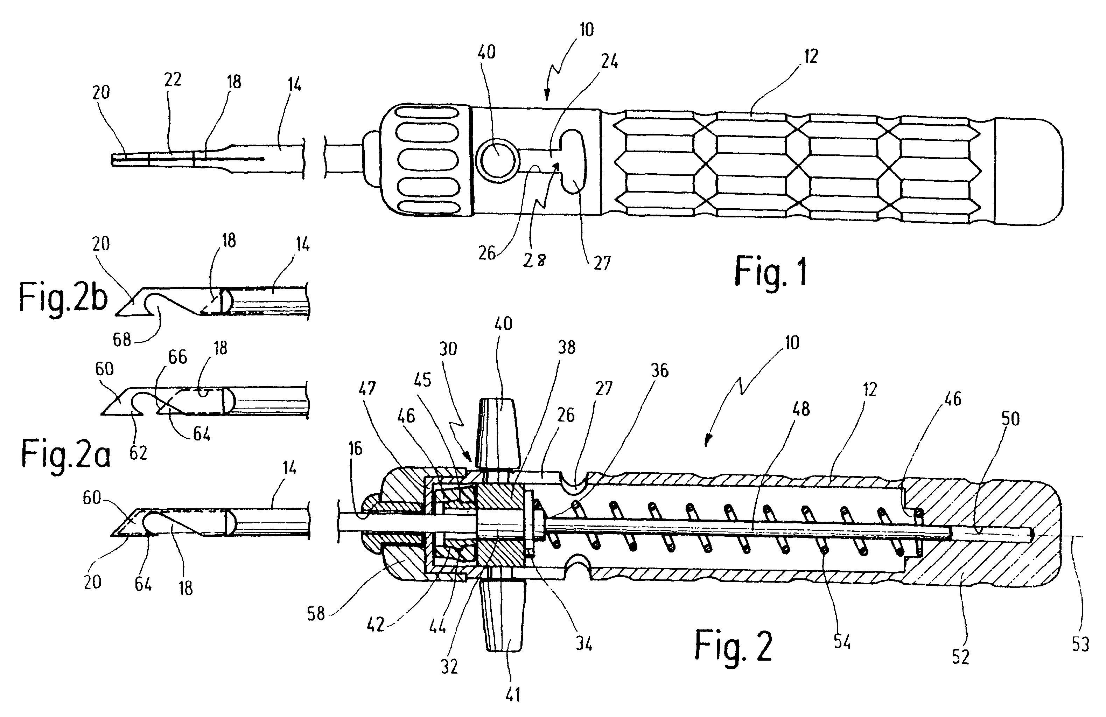

Der Betätigungsmechanismus 30 (siehe insbesondere Schnittdarstellung von Fig. 2) weist ein zentrales zylindrisches Stück 32 auf, das längs der Mittellängsachse des Gehäuses 12 angeordnet ist. Das zylindrische Stück 32 ist distal mit dem Betätigungselement 16 verbunden.

Proximal springt vom zylindrischen Stück 32 ein radialer Ringflansch 34 vor, vom Ringflansch 34 springt axial ein Zapfen 36 vor.

Über das zylindrische Stück ist eine Hülse 38 geschoben, die am Ringflansch 34 zum Liegen kommt.

Der lichte Innendurchmesser der Hülse 38 ist derart, daß sie an der Außenseite des zylindrischen Stückes 32 anliegt, um dieses aber verdrehbar ist.

Von der Hülse 38 springen diametral gegenüberliegend, radial zwei Betätigungsstifte 40 und 41 vor, die durch entsprechend diametral gegenüberliegende T-Schlitzöffnungen 26 der Bajonettführung 24 hindurchreichen.

Von der Hülse 38 springt distal ein Rohrflansch 42 vor, der eine umfängliche Schulter 44 aufweist.

Über den Rohrflansch 42 ist ein hohlzylindrisches Dämpfungsglied 45 in Form eines elastischen Elementes 46 geschoben, das aus einem elastischen Material hergestellt ist.

Die Schulter 44 ist so ausgebildet, daß ein Aufschieben des elastischen Elements 46 auf den Rohrflansch 42 möglich ist, ein Abziehen jedoch gesperrt wird. Proximal liegt das ringförmige Ende des Dämpfungsgliedes 46 an einer Schulter zwischen Hülse 38 und durchmessergeringerem Rohrflansch 42 an.

Die axiale Länge des Dämpfungsgliedes 45 ist länger als die Länge des Rohrflansches 42, so daß distal das rohrförmige elastische Element 46 über den Rohrflansch 42 hinaussteht.

Der vom Ringflansch 34 proximal vorstehende Zapfen 36 ist mit einer Stange 48 verbunden, die durch den hohlen Innenraum des Gehäuses 12 bis in ein Sackloch 50 am proximalen Endstück 52 des Gehäuses 12 hineinreicht. In der in Fig. 2 dargestellten Stellung reicht die Stange 48 nur etwas in das Sackloch 50 hinein, das zugleich als Führungszylinder für die Stange 48 dient. Das Sackloch 50 mündet in einer etwas breiteren Vertiefung 46 in Richtung Innenraum des Gehäuses 12. Im Innenraum ist um die Stange 48 eine Schraubenfeder 54 angeordnet, deren eines Ende sich in der Vertiefung 46 im Endstück 52 und deren anderes Ende auf dem Ringflansch 34 abgestützt ist. Die Feder 54 ist leicht auf Druck vorgespannt, schiebt somit den Betätigungsmechanismus 30 in die in Fig. 1 und 2 dargestellte Stellung. In dieser Stellung wird das distale Ende des Dämpfungsgliedes 45 gegen eine stirnseitig abschließende Wand 47 des Gehäuses 12 gedrückt.

Eine Mutter 58 dient dazu, den Schaft 14 mit dem Gehäuse 12 zu verbinden.

Aus Fig. 2 ist zu entnehmen, daß der Amboß 20 als Haken 60 ausgebildet ist, wobei eine Spitze 62 des Hakens 60 einer Spitze 64 der Klinge 18 zugewandt ist.

Im Bereich des Amboß 20 ist eine Ausnehmung 68 ausgespart, um darin den zu schneidenden Faden einzulegen. Der Haken 60 erleichtert ein Einfangen und Einlegen des Fadens.

Zum Durchschneiden eines Fadens wird der Fadenschneider 10 über das Gehäuse 12 ergriffen, und über zwei Finger werden die Betätigungsstifte 40 und 41 entgegen der Kraft der Feder 54 von distal nach proximal bewegt und dabei in der Bajonettführung 24 geführt. Wurden die Betätigungsstifte 40 und 41 bis auf Höhe des Querbereiches 27 der T-Schlitzöffnung 26 zurückgezogen, werden diese etwas umfänglich verdreht und treten und einen der Querbereiche 27 ein. Dies wurde durch die freie Drehbarkeit der Hülse 38 relativ zum lediglich axial verschiebbaren zylindrischen Stück 32 erreicht. Dessen Verdrehen wird durch eine entsprechende mechanische Sperre oder dgl. verhindert. In dieser Stellung ist der Betätigungsmechanismus 30 gespannt und arretiert. Die Klinge 18 wurde dabei soweit vom Amboß 20 weg bewegt, wie das in Fig. 2b dargestellt ist. Nunmehr kann der Faden in den Haken 60 des Amboß 20 eingelegt werden. Durch leichtes Antippen der Betätigungsstifte 40, 41 in umfänglicher Richtung werden diese in den mittigen Bereich der T-Schlitzöffnung 26 verschoben und die relativ starke Kraft der gespannten Feder 54 sorgt dafür, daß der Betätigungsmechanismus 30 schlagartig, also guillotineartig nach distal bewegt wird. Wie aus der Rückabfolge der Figuren 2b, 2a zu 2 zu sehen ist, bewegt sich dabei die Klinge 18 bzw. deren Spitze 64 sehr rasch auf den im Haken 60 des Amboß 20 gefangenen Faden (hier nicht dargestellt) zu und trennt diesen sofort durch. Der Aufprall des Betätigungsmechanismus 30 auf die Innenseite der Wand 47 wird durch Stauchen des Dämpfungsgliedes 45 gedämpft.

Der beschriebene Fadenschneider 10 ist als Einzelinstrument aufgebaut. Er kann auch als Bestandteil eines multifunktionalen Instruments ausgebildet sein, das nicht nur den Fadenschneider enthält sondern auch eine Nähvorrichtung aufweist.

## Patentansprüche

1. Chirurgischer Fadenschneider, mit einer über einen Betätigungsmechanismus (30) relativ auf einen Amboß (20) hin und von diesem wegbewegbaren Klinge (18), sowie mit einer Feder (54), über die die Klinge (18) in einer Bewegungsrichtung mit Federkraft beaufschlagbar ist, **dadurch gekennzeichnet, daß** die Feder (54) die Klinge (18) in Schneiderichtung beaufschlagt, daß eine Arretierung (28) vorgesehen ist, über die die Klinge (18) in einer vom Amboß (20) entfernten Position feststellbar ist, und daß die Kraft der Feder (54) derart eingestellt ist, daß die Klinge (18), nach Lösen der Arretierung (28), guillotineartig auf den Amboß (20) zu bewegbar ist.

2. Chirurgischer Fadenschneider nach Anspruch 1, **dadurch gekennzeichnet, daß** der Betätigungsmechanismus (30) längs der Bewegungsrichtung der Klinge (18) bewegbar ist, und relativ zur Bewegungsrichtung drehbar und in einer verdrehten Stellung durch die Arretierung (28) arretierbar ist.

3. Chirurgischer Fadenschneider nach Anspruch 1 oder 2, **dadurch gekennzeichnet, daß** der Betätigungsmechanismus (30) proximal in einem griffartigen Gehäuse (12) aufgenommen ist.

4. Chirurgischer Fadenschneider nach Anspruch 3, **dadurch gekennzeichnet, daß** die Arretierung (28) zumindest ein vom Betätigungsmechanismus (30) radial vorspringendes Betätigungselement (40, 41) aufweist, das durch eine Bajonettführung (24) im Gehäuse (12) reicht.

5. Chirurgischer Fadenschneider nach Anspruch 4, **dadurch gekennzeichnet, daß** zwei diametral gegenüberliegende, radial vorspringende Betätigungsstifte (40, 41) vorgesehen sind.

6. Chirurgischer Fadenschneider nach Anspruch 4 oder 5, **dadurch gekennzeichnet, daß** die Bajonettführung (24) als T-förmige Schlitzöffnung (26) im Gehäuse (12) ausgebildet ist.

7. Chirurgischer Fadenschneider nach Anspruch 6, **dadurch gekennzeichnet, daß** Querbereiche (27) der T-förmigen Schlitzöffnung (26), die sich in umfänglicher Richtung des Gehäuses (12) erstrecken, in Richtung des Amboß (20) etwas geneigt sind.

8. Chirurgischer Fadenschneider nach einem der Ansprüche 3 bis 7, **dadurch gekennzeichnet, daß** die Feder (54) im griffartigen Gehäuse (12) angeordnet ist.

9. Chirurgischer Fadenschneider nach einem der Ansprüche 1 bis 8, **dadurch gekennzeichnet, daß** ein Dämpfungsglied (45) vorgesehen ist, das die Bewegung der Klinge (18) am Ende der Schneidebewegung dämpft.

10. Chirurgischer Fadenschneider nach Anspruch 9, **dadurch gekennzeichnet, daß** der Betätigungsmechanismus (30) das Dämpfungsglied (45) aufweist, durch das ein Aufprall des Betätigungsmechanismus (30) gegen einen Anschlag (47) am Ende der Schneidebewegung gedämpft wird.

11. Chirurgischer Fadenschneider nach Anspruch 9, **dadurch gekennzeichnet, daß** im Gehäuse (12) ein elastisches Dämpfungsglied (45) vorgesehen ist, das gegen eine Wand (47) des Gehäuses prallt und dabei verformbar ist.

12. Chirurgischer Fadenschneider nach einem der Ansprüche 1 bis 11, **dadurch gekennzeichnet, daß** die Klinge (18) in einem Schaft (14) aufgenommen ist, und daß Schaft (14) und Klinge (18) voneinander lösbar sind.

13. Chirurgischer Fadenschneider nach einem der Ansprüche 1 bis 12, **dadurch gekennzeichnet, daß** der Amboß (20) die Form eines Hakens (60) aufweist.

14. Chirurgischer Fadenschneider nach Anspruch 13, **dadurch gekennzeichnet, daß** eine Spitze (62) des Hakens (60) einer Spitze (64) der Klinge (18) zugewandt ist.

15. Chirurgischer Fadenschneider nach einem der Ansprüche 12 bis 14, **dadurch gekennzeichnet, daß** der Amboß (20) Teil des Schaftes (14) ist, und daß der Schaft (14) mit dem griffartigen Gehäuse (12) lösbar verbindbar ist.

16. Chirurgischer Fadenschneider nach einem der Ansprüche 1 bis 15, **dadurch gekennzeichnet, daß** der Amboß (20) einen Längsschlitz (22) aufweist, in den die Klinge (18) einfahrbar ist.

17. Chirurgischer Fadenschneider nach einem der Ansprüche 1 bis 15, **dadurch gekennzeichnet, daß** die Klinge auf eine Fläche des Amboß aufläuft.

18. Chirurgischer Fadenschneider nach einem der Ansprüche 1 bis 15, **dadurch gekennzeichnet, daß** die Klinge eine Schneidekante aufweist, die mit einer Kante des Amboß zusammenwirkt.

19. Chirurgischer Fadenschneider nach einem der Ansprüche 1 bis 18, **dadurch gekennzeichnet, daß** die Länge des Laufweges der Klinge einstellbar ist.

20. Chirurgischer Fadenschneider nach einem der Ansprüche 1 bis 19, **dadurch gekennzeichnet, daß** die Feder als Schraubenfeder, Luftdruckfeder, magnetisch gespannte Feder ausgebildet ist.

21. Chirurgischer Fadenschneider nach einem der Ansprüche 1 bis 20, **dadurch gekennzeichnet, daß** er Teil eines multifunktionalen Instrumentes ist.

## Claims

1. Surgical thread cutter having a blade (18) movable via an actuation mechanism (30) relative to an anvil (20) toward and away from the latter, and having a spring (54) by way of which the blade (18) can be acted upon by spring force in one movement direction, **characterized in that** the spring (54) acts upon the blade (18) in the cutting direction, a retention system (28) is provided by means of which the blade (18) can be locked in a position remote from the anvil (20), and the force of the spring (54) is set in such a way that after the retention system (28) is released, the blade (18) can be moved onto the anvil (20) in the manner of a guillotine.

2. Surgical thread cutter of Claim 1, **characterized in that** the actuation mechanism (30) is movable along the movement direction of the blade (18), and is rotatable relative to the movement direction and can be retained by the retention system (28) in a rotated position.

3. Surgical thread cutter of Claims 1 or 2, **characterized in that** the actuation mechanism (30) is received proximally in a handle-like housing (12).

4. Surgical thread cutter of Claim 3, **characterized in that** the retention mechanism (28) has at least one actuation element (40, 41), projecting radially from the actuation mechanism (30), which extends through a bayonet guide (24) in the housing (12).

5. Surgical thread cutter of Claim 4, **characterized in that** two diametrically opposite, radially projecting actuation pins (40, 41) are provided.

6. Surgical thread cutter of Claims 4 or 5, **characterized in that** the bayonet guide (24) is configured as a T-shaped slot opening (26) in the housing (12).

7. Surgical thread cutter of Claim 6, **characterized in that** the transverse regions (27) of the T-shaped slot openings (26), which extend in the circumferential direction of the housing (12), are inclined somewhat in the direction of the anvil (20).

8. Surgical thread cutter of anyone of Claims 3 through 7, **characterized in that** the spring (54) is arranged in the handle-like housing (12).

9. Surgical thread cutter of anyone of Claims 1 through 8, **characterized in that** a damping member (45) is provided which damps the movement of the blade (18) at the end of the cutting movement.

10. Surgical thread cutter of Claim 9, **characterized in that** the actuation mechanism (30) has the damping member (45) which damps an impact of the actuation mechanism (30) against a stop (47) at the end of the cutting movement.

11. Surgical thread cutter of Claim 9, **characterized in that** there is provided in the housing (12) an elastic damping member (45) which strikes against a wall (47) of the housing and **in that** context is deformable.

12. Surgical thread cutter of anyone of Claims 1 through 11, **characterized in that** the blade (18) is received in a shaft (14), and **in that** the shaft (14) and the blade (18) are detachable from one another.

13. Surgical thread cutter of anyone of Claims 1 through 12, **characterized in that** the anvil (20) is in the shape of a hook (60).

14. Surgical thread cutter of Claim 13, **characterized in that** a tip (62) of the hook (60) faces toward a tip (64) of the blade (18).

15. Surgical thread cutter of anyone of Claims 12 through 14, **characterized in that** the anvil (20) is part of the shaft (14), and **in that** the shaft (14) can be detachably joined to the handle-like housing (12).

16. Surgical thread cutter of anyone of Claims 1 through 15, **characterized in that** the anvil (20) has a longitudinal slot (22) into which the blade (18) can enter.

17. Surgical thread cutter of anyone of Claims 1 through 15, **characterized in that** the blade runs along a surface of the anvil.

18. Surgical thread cutter of anyone of Claims 1 through 15, **characterized in that** the blade has a cutting edge which coacts with an edge of the anvil.

19. Surgical thread cutter of anyone of Claims 1 through 18, **characterized in that** the length of the travel of the blade is adjustable.

20. Surgical thread cutter of anyone of Claims 1 through 19, **characterized in that** the spring is configured as a helical spring, as a compressed-air spring, or as a magnetically cocked spring.

21. Surgical thread cutter of anyone of Claims 1 through 20, **characterized in that** it is part of a multifunctional instrument.

## Revendications

1. Coupe-fils chirurgical comportant une lame (18) déplaçable, au moyen d'un mécanisme d'actionnement (30), vers une enclume (20) et depuis celle-ci, ainsi qu'un ressort (54) par lequel la lame (18) est soumise à la force d'un ressort dans une direction de déplacement, **caractérisé en ce que** le ressort (54) agit sur la lame (18) dans la direction de coupe, **en ce qu'**il est prévu un dispositif d'arrêt (28) par lequel la lame (18) peut être fixée dans une position éloignée de l'enclume (20), et **en ce que** la force du ressort (54) est réglée de manière qu'après déverrouillage du dispositif d'arrêt (28), la lame (18) puisse être déplacée vers l'enclume (20) à la manière d'une guillotine.

2. Coupe-fils chirurgical selon la revendication 1, **caractérisé en ce que** le mécanisme d'actionnement (30) est déplaçable le long de la direction de déplacement de la lame (18), et peut tourner par rapport à la direction de déplacement et peut être bloqué dans une position tournée, sous l'effet du dispositif d'arrêt (28).

3. Coupe-fils chirurgical selon la revendication 1 ou 2, **caractérisé en ce que** le mécanisme d'actionnement (30) est logé du côté proximal dans un boîtier (12) de type poignée.

4. Coupe-fils chirurgical selon la revendication 3, **caractérisé en ce que** le dispositif d'arrêt (28) comporte au moins un élément d'actionnement (40, 41) faisant saillie radialement du mécanisme d'actionnement (30) et qui s'étend vers l'intérieur du boîtier (12) en passant par un guide à baïonnette (24).

5. Coupe-fils chirurgical selon la revendication 4, **caractérisé en ce qu'**il est prévu deux broches d'actionnement (40, 41) faisant saillie radialement, diamétralement opposées.

6. Coupe-fils chirurgical selon la revendication 4 ou 5, **caractérisé en ce que** le guide à baïonnette (24) est réalisé en tant qu'ouverture en fente (26) en forme de T dans le boîtier (12).

7. Coupe-fils chirurgical selon la revendication 6, **caractérisé en ce que** des zones transversales (27) de l'ouverture en fente (26) en forme de T, qui s'étendent dans la direction périphérique du boîtier (12), sont légèrement inclinées en direction de l'enclume (20).

8. Coupe-fils chirurgical selon l'une des revendications 3 à 7, **caractérisé en ce que** le ressort (54) est disposé dans le boîtier (12) de type poignée.

9. Coupe-fils chirurgical selon l'une des revendications 1 à 8, **caractérisé en ce qu'**il est prévu un organe d'amortissement (45) qui amortit le mouvement de la lame (18) à la fin du mouvement de coupe.

10. Coupe-fils chirurgical selon la revendication 9, **caractérisé en ce que** le mécanisme d'actionnement (30) comporte l'organe d'amortissement (45) par lequel un rebondissement du mécanisme d'actionnement (30) contre une butée (47) est amorti à la fin du mouvement de coupe.

11. Coupe-fils chirurgical selon la revendication 9, **caractérisé en ce que** dans le boîtier (12) est prévu un organe d'amortissement (45) élastique qui rebondit contre une paroi (47) du boîtier et est ainsi déformable.

12. Coupe-fils chirurgical selon l'une des revendications 1 à 11, **caractérisé en ce que** la lame (18) est reçue dans une tige (14) et **en ce que** la tige (14) et la lame (18) peuvent être séparées l'une de l'autre.

13. Coupe-fils chirurgical selon l'une des revendications 1 à 12, **caractérisé en ce que** l'enclume (20) présente la forme d'un crochet (60).

14. Coupe-fils chirurgical selon la revendication 13, **caractérisé en ce qu'**une pointe (62) du crochet (60) est tournée vers une pointe (64) de la lame (18).

15. Coupe-fils chirurgical selon l'une des revendications 12 à 14, **caractérisé en ce que** l'enclume (20) fait partie de la tige (14) et **en ce que** la tige (14) peut être reliée de manière séparable au boîtier (12) de type poignée.

16. Coupe-fils chirurgical selon l'une des revendications 1 à 15, **caractérisé en ce que** l'enclume (20) présente une fente longitudinale (22) dans laquelle peut être introduite la lame (8).

17. Coupe-fils chirurgical selon l'une des revendications 1 à 15, **caractérisé en ce que** la lame passe sur une surface de l'enclume.

18. Coupe-fils chirurgical selon l'une des revendications 1 à 15, **caractérisé en ce que** la lame présente une arête de coupe qui coopère avec une arête de l'enclume.

19. Coupe-fils chirurgical selon l'une des revendications 1 à 18, **caractérisé en ce que** la longueur de la course de la lame est réglable.

20. Coupe-fils chirurgical selon l'une des revendications 1 à 19, **caractérisé en ce que** le ressort est réalisé en tant que ressort hélicoïdal, ressort pneumatique, ressort tendu magnétiquement.

21. Coupe-fils chirurgical selon l'une des revendications 1 à 20, **caractérisé en ce qu'**il fait partie d'un instrument multifonctionnel.
